# EUROPEAN PATENT SPECIFICATION

(11) **EP 3 049 060 B1**
(45) Date of publication and mention of the grant of the patent: **25.10.2017**
(21) Application number: 14772547.7
(22) Date of filing: 15.09.2014
(51) Int. Cl.: A61K 9/08, A61K 47/02, A61K 47/38, A61K 9/00

(54) **COMPOSITION FOR APPLICATION TO A MUCOSA COMPRISING A CELLULOSE ETHER**
ZUSAMMENSETZUNG ZUR ANWENDUNG AUF EINER SCHLEIMHAUT MIT EINEM CELLULOSEETHER
COMPOSITION POUR APPLICATION À UNE MUQUEUSE COMPRENANT UN ÉTHER DE CELLULOSE

(30) Priority: 25.09.2013 US 201361882041 P
(43) Date of publication of application: 03.08.2016
(73) Proprietor: Dow Global Technologies LLC, Midland, MI 48674 (US)
(72) Inventor: CURTIS-FISK, Jaime L., Freeland, MI 48623 (US); JORDAN, Susan L., Doylestown, PA 18902 (US); ROGERS, True L., Midland, MI 48642 (US); KNARR, Matthias, 31582 Nienburg/Weser (DE); BRACKHAGEN, Meinolf, 29664 Walsrode (DE); ADDEN, Roland, 29664 Walsrode (DE)
(74) Representative: f & e patent
(86) International application number: PCT/US2014/055568
(87) International publication number: WO 2015/047762

(56) References cited:
- WO-A1-99/38492
- WO-A1-2012/051035

## Description

### FIELD

The present invention concerns a composition for application to a mucosa, e.g. for transmucosal delivery of a physiologically active agent, and a method of administering a physiologically active agent to an individual.

### INTRODUCTION

Compositions for application to mucosae, such as pharmaceutical compositions for transmucosal delivery of physiologically active agents, have been known for a long time. Nasal drops and sprays have been known as drug delivery systems intended for administration to the nasal cavity. However, known nasal drops and sprays often rapidly exit the nasal cavity either via dripping from the nostrils or via the back of the nasal cavity into the nasopharynx, which can lead to insufficient efficacy of the physiologically active agent(s). High-viscosity delivery systems, such as ointments or gels, are retained in the nasal cavity for a longer time period, but the exact dosage of ointments and gels is difficult to meter and subsequently deliver to the desired location within the nasal cavity. Similar problems are experienced if pharmaceutical compositions are applied to other mucosae, such as the mucous membrane of the eyes or to mucosae in the oral cavity, such as the buccal mucosa.

To address this problem, European Patent EP 0 023 359 discloses a powdery pharmaceutical composition for application to the mucosa of the nasal cavity which comprises a drug and a carrier. At least 90% of the composition consists of particles having an effective particle diameter of 20 to 250 µm. The composition comprising a lower alkyl ether of cellulose having a viscosity, determined at 37°C±0.2°C for a 2% aqueous solution thereof, of 5 to 5000 mPa•s. The lower alkyl ether of cellulose is preferably methyl cellulose, hydroxypropyl cellulose or hydroxypropyl methylcellulose. Those having a degree of ether substitution of 0.1 to 6, especially 0.4 to 4.6 are said to be preferred. The pharmaceutical composition absorbs mucus on the nasal mucosa and covers the nasal mucosa as a fluid surface. Unfortunately, spraying a powdery pharmaceutical composition into the nasal cavity is quite complex. The European Patent EP 0 023 359 suggests filling a capsule with the powdery composition, mounting it in a sprayer equipped by a needle, piercing the capsule with the needle to provide minute holes on the top and bottom sides of the capsule and thereafter sending air by means of a rubber ball to jet out the powder. Moreover, a powdery composition often gives the feel of foreign matter in the nasal cavity, irritates the nasal cavity and can lead to drying out of the nasal cavity.

WO 99/38492 A1 discloses a nasal drop composition comprising the vasoconstrictor Xylometazoline Hydrochloride, Hydroxypropyl methyl cellulose, sorbitol and water. In view of the above-mentioned deficiencies of the prior art compositions to be applied to a mucosa, the object of the present invention is to provide a composition that can be easily applied onto a mucosa, and that is retained on the mucosa for an extended time period.

### SUMMARY

One aspect of the present invention is a composition for application to a mucosa which comprises
i) from 0.1 to 10 weight percent of a tonicity-adjusting agent, based on the total weight of the composition,
ii) a liquid diluent of which at least 55 weight percent is water, and
iii) from 0.1 to 6 weight percent of a cellulose ether, based on the total weight of the composition,
wherein the cellulose ether has a viscosity from 1.2 to 8000 mPa•s, measured as 2 wt. % aqueous solution at 20 °C at a shear rate of 10 s⁻¹, and
wherein the cellulose ether has anhydroglucose units joined by 1-4 linkages and has methyl groups, hydroxyalkyl groups, and optionally alkyl groups being different from methyl as substituents such that
hydroxyl groups of anhydroglucose units are substituted with methyl groups such that s23/s26 is 0.29 or less,
wherein s23 is the molar fraction of anhydroglucose units wherein only the two hydroxyl groups in the 2- and 3-positions of the anhydroglucose unit are substituted with a methyl group and
wherein s26 is the molar fraction of anhydroglucose units wherein only the two hydroxyl groups in the 2- and 6-positions of the anhydroglucose unit are substituted with a methyl group.

Another aspect of the present invention is a container which comprises the above-mentioned composition, wherein the container is designed for releasing the composition by spraying or as drops.

Yet another aspect of the present invention is a method of transmucosal administration of a physiologically active agent to an individual wherein the above-mentioned composition, that additionally comprises a physiologically active agent, is applied to a mucosa of the individual.

### DETAILED DESCRIPTION (DESCRIPTION OF EMBODIMENTS)

Surprisingly, it has been found that the composition of the present invention exhibits a gelation temperature of up to 37 °C, typically up to 35 °C, and more typically up to 33 °C. The gelation temperature of the composition of the present invention is generally at least 18 °C, typically at least 21 °C, more typically at least 24 °C, and most typically at least 27 °C.

The composition of the present invention is very useful for application to a mucosa, e.g. for transmucosal delivery of a physiologically active agent. A low viscosity at 5°C or 20 °C, i.e., at a temperature at which the composition is usually stored and/or applied, facilitates the release of the composition from a container comprising such composition, e.g. as drops or by spraying, and the administration of the composition to a mucosa. The temperature of the composition increases after its application to a mucosa.

Thermal gelation at the gelation temperature of the composition of the present invention triggers two phenomena to maximize efficacy of the delivery system: 1) The high-viscosity major portion of the thermally gelled composition facilitates retention of the composition of the present invention on the mucosa; 2) syneresis occurs upon thermal gelation of the composition of the present invention, and the hydroxyalkyl methylcellulose, the tonicity-adjusting agent and the typically present physiologically active agent are concentrated into a syneresed layer most intimately interfacing with the mucosa instead of being rinsed off the mucosa in syneresis fluid.

Conventionally, hydroxyalkyl methylcellulose, particularly hydroxypropyl methylcellulose, has been found to be very useful in a variety of applications, providing thickening, freeze/thaw stability, lubricity, moisture retention and release, film formation, modified-release, texture, consistency, shape retention, emulsification, binding, gelation, and suspension properties. However, hydroxyalkyl methylcelluloses, such as hydroxypropyl methylcellulose, usually do not provide a sufficient thickening effect to compositions at temperatures encountered within the nasal cavities of mammals, such as those of human beings, as shown in the accompanying examples. One unusual property of hydroxyalkyl methylcelluloses is that they are known to exhibit reverse thermal gelation in water; in other words, hydroxyalkyl methylcellulose gels at temperatures above 50 °C when dissolved at a concentration of 2% and forms a liquid if cooled again down to temperatures of 20 °C or less. Most grades of hydroxyalkyl methylcellulose, dissolved alone to 2 wt.% in water at about 5 °C, will precipitate and subsequently gel at least about 10 °C higher than the normal body temperature of a human being.

In the present invention, the composition for application to a mucosa comprises at least one cellulose ether, which has anhydroglucose units joined by 1-4 linkages and which has methyl groups, hydroxyalkyl groups, and optionally alkyl groups being different from methyl as substituents. The hydroxyalkyl groups can be the same or different from each other. Preferably the cellulose ether comprises one or two kinds of hydroxyalkyl groups, more preferably one or more kinds of hydroxy-C₁₋₃-alkyl groups, such as hydroxypropyl and/or hydroxyethyl. Useful optional alkyl groups are, e.g., ethyl or propyl, ethyl being preferred. Preferred ternary cellulose ethers are ethyl hydroxypropyl methyl celluloses, ethyl hydroxyethyl methyl celluloses, or hydroxyethyl hydroxypropyl methyl celluloses. Preferred cellulose ethers are hydroxyalkyl methyl celluloses, particularly hydroxy-C₁₋₃-alkyl methyl celluloses, such as hydroxypropyl methylcelluloses or hydroxyethyl methylcelluloses.

An essential feature of the cellulose ether is its unique distribution of methyl groups on the anhydroglucose units such that s23/s26 is 0.29 or less, preferably 0.28 or less, more preferably 0.26 or less, most preferably 0.24 or less, and particularly 0.22 or less. Typically s23/s26 is 0.05 or more, more typically 0.08 or more, and most typically 0.11 or more.

In the ratio s23/s26, s23 is the molar fraction of anhydroglucose units wherein only the two hydroxyl groups in the 2- and 3-positions of the anhydroglucose unit are substituted with methyl groups and s26 is the molar fraction of anhydroglucose units wherein only the two hydroxyl groups in the 2- and 6-positions of the anhydroglucose unit are substituted with methyl groups. For determining the s23, the term "the molar fraction of anhydroglucose units wherein only the two hydroxyl groups in the 2- and 3-positions of the anhydroglucose unit are substituted with methyl groups" means that the 6-positions are not substituted with methyl; for example, they can be unsubstituted hydroxyl groups or they can be substituted with hydroxyalkyl groups, methylated hydroxyalkyl groups, alkyl groups different from methyl or alkylated hydroxyalkyl groups. For determining the s26, the term "the molar fraction of anhydroglucose units wherein only the two hydroxyl groups in the 2- and 6-positions of the anhydroglucose unit are substituted with methyl groups" means that the 3-positions are not substituted with methyl; for example, they can be unsubstituted hydroxyl groups or they can be substituted with hydroxyalkyl groups, methylated hydroxyalkyl groups, alkyl groups different from methyl or alkylated hydroxyalkyl groups.

The term "hydroxyl group substituted with methyl group" or "hydroxyl group substituted with hydroxyalkyl group" as used herein means that the hydrogen atom on the hydroxyl group is replaced by a methyl group or a hydroxyalkyl group.

Formula I below illustrates the numbering of the hydroxyl groups in anhydroglucose units. Formula I is only used for illustrative purposes and does not represent the cellulose ethers of the invention; the substitution with hydroxyalkyl groups is not shown in Formula I.

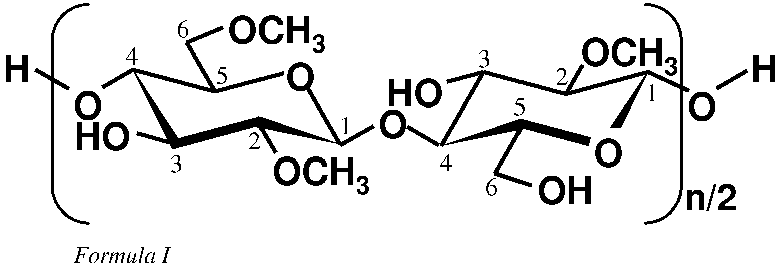

The cellulose ether preferably has a DS(methyl) of from 1.6 to 2.5, more preferably from 1.7 to 2.4, and most preferably from 1.7 to 2.2. The degree of the methyl substitution, DS(methyl), of a cellulose ether is the average number of OH groups substituted with methyl groups per anhydroglucose unit. For determining the DS(methyl), the term "OH groups substituted with methyl groups" does not only include the methylated OH groups directly bound to the carbon atoms of the cellulose backbone but also methylated OH groups that have been formed after hydroxyalkylation.

The cellulose ether generally has an MS(hydroxyalkyl) of 0.05 to 0.35, preferably 0.05 to 0.33, more preferably 0.05 to 0.29, and most preferably 0.05 to 0.25 or 0.05 to 0.20 or even 0.05 to 0.15. The degree of the hydroxyalkyl substitution is described by the MS (molar substitution). The MS(hydroxyalkyl) is the average number of hydroxyalkyl groups which are bound by an ether bond per mole of anhydroglucose unit. During the hydroxyalkylation, multiple substitutions can result in side chains.

The degree of substitution of methoxyl groups (DS) and the molar substitution of hydroxyalkoxyl groups (MS) can be determined by Zeisel cleavage of the hydroxyalkyl methylcellulose with hydrogen iodide and subsequent quantitative gas chromatographic analysis (G. Bartelmus and R. Ketterer, Z. Anal. Chem., 286 (1977) 161-190). When the hydroxyalkyl methylcellulose is hydroxypropyl methylcellulose, the determination of the % methoxyl and % hydroxypropoxyl is carried out according to the United States Pharmacopeia (USP 35, "Hypromellose", pages 3467-3469). The values obtained are % methoxyl and % hydroxypropoxyl. These are subsequently converted into degree of substitution (DS) for methoxyl substituents and molar substitution (MS) for hydroxypropoxyl substituents. Residual amounts of salt have been taken into account in the conversion.

The cellulose ether utilized in the composition of the present invention has a viscosity of from 1.2 to 8000 mPa•s, preferably from 1.8 to 6000 mPa•s, more preferably from 2.4 to 3000 mPa•s, even more preferably from 2.4 to 1000 mPa·s, and most preferably 3.0 to 500 mPa·s, measured as a 2 weight-% solution in water at 20 °C at a shear rate of 10 s⁻¹. The viscosity of the cellulose ether is measured as a 2 weight-% solution in water at 20 °C at a shear rate of 10 s⁻¹ with an Anton Paar Physica MCR 501 rheometer and cup and bob fixtures (CC-27).

Methods of making the cellulose ethers utilized in the composition of the present invention are described in detail in the Examples. Methods of making the cellulose ethers are also described in the published International Patent Applications WO2012/051035 and WO2012/051034. Another essential ingredient of the composition of the present invention is a tonicity-adjusting agent. One or more tonicity-adjusting agents may be included in the composition of the present invention to partially or fully achieve tonicity with body fluids, e.g. fluids of the nasal cavity or fluids of the eye, resulting in reduced levels of irritation. In one aspect of the present invention the tonicity-adjusting agent is an alkali or alkaline earth metal halide, preferably an alkali or alkaline earth metal chloride. Examples of pharmaceutically acceptable tonicity-adjusting agents include, but are not limited to, sodium chloride, potassium chloride, dextrose, xylitol, calcium chloride, glucose, glycerin, mannitol, and sorbitol. A tonicity-adjusting agent is preferably included in an amount of from 0.1 to 10 percent, more preferably from 0.2 to 8.0 percent, even more preferably from 0.3 to 6.0 percent, or from 0.5 to 4.0 percent, and most preferably from 0.5 to 2.0 percent, based on the total weight of the composition. In one embodiment, the tonicity-adjusting agent is dextrose and/or xylitol. In another embodiment, the tonicity-adjusting agent is sodium chloride. In yet another embodiment the tonicity-adjusting agent is a buffering agent. Suitable buffering agents include, but are not limited to, organic acid salts such as salts of citric acid, gluconic acid, carbonic acid, tartaric acid, succinic acid, acetic acid, phthalic acid, or phosphoric acid. A phosphate buffer is particularly useful. Phosphate buffers typically comprise sodium or potassium phosphate dibasic or sodium or potassium phosphate monobasic. In addition, amino acid components can also be used as buffering agent. Such amino acid component includes without limitation glycine and histidine. The buffering agent provides improved pH control. In one embodiment, the composition of the invention has a pH between 5.0 and 8.0, preferably between 6.0 and 8.0, and more preferably between 6.0 and 7.0. In a specific embodiment, a composition of the invention has a pH of about 6.5.

It has surprisingly been found that a composition of the present invention which comprises the cellulose ether described further above in combination with a tonicity-adjusting agent exhibits thermal gelation at a lower temperature than a comparable composition containing the same type and amount of cellulose ether without the tonicity-adjusting agent. Alternatively, a lower concentration of the afore-mentioned cellulose ether can be utilized in the presence of a tonicity-adjusting agent while still achieving thermal gelation of the composition at the desired temperature. The effects of the tonicity-adjusting agent in combination with the cellulose ether described further above are illustrated in more detail in the Examples.

The composition of the present invention is useful for application to mucosae, e.g., for intranasal, buccal, sublingual, vaginal, ocular or rectal application.

In one embodiment of the invention the composition comprises one or more physiologically active agents, preferably one or more drugs, one or more diagnostic agents, or one or more essential oils, or one or more physiologically active agents which are useful for cosmetic or nutritional purposes. The term "drug" denotes a compound having beneficial prophylactic and/or therapeutic properties when administered to an individual, typically a mammal, especially a human individual. Physiologically active agents that are useful for transmucosal delivery, such as intranasal, buccal, sublingual, vaginal, ocular or rectal delivery, or delivery through a mucosal membrane located on the gums or lips are known in the art.

The composition of the present invention is particularly useful for intranasal delivery of one or more physiologically active agents or for delivery through a mucosal membrane located in the oral cavity, such as drugs utilized in therapies for allergic rhinitis, nasal congestion and infections, in treatments of diabetes, migraine, nausea, smoking cessation, acute pain relief, nocturnal enuresis, osteoporosis, vitamin B-12 deficiency and for administering intranasal vaccine, however the physiologically active agents are not limited to these examples. Especially preferred drugs are acetaminophen, azelastine hydrochloride, beclomethasone dipropionate monohydrate, sumatriptan succinate, dihydroergotamine mesylate, fluticasone propionate, triamcinolone acetonide, budesonide, fentanyl citrate, butorphanol tartrate, zolmitriptan, desmopressin acetate hydrate, salmon calcitonin, nafarelin acetate, buserelin acetate, elcatonin, oxytocin, insulin, mometasone furoate, estradiol, metoclopramide, xylometazoline hydrochloride, ipratropium bromide hydrate, olopatadine hydrochloride, oxymetazoline hydrochloride, dexpanthenol, hydrocortisone, naphazoline hydrochloride, phenylephrine hydrochloride, mepyramine maleate, phenylephrine hydrochloride, cromolyn sodium, levocabastine hydrochloride, vitamin B 12, prednisolone sodium metasulphobenzoate, naphazoline nitrate, tetrahydrozoline hydrochloride, chlorpheniramine maleate, benzethonium chloride, ketotifen fumarate, histamine dihydrochloride, fusafungine, or combinations thereof. Examples of essential oils are menthol, methyl salicylate, thymol, eucalyptus oil, camphor, anise, sweet orange, or combinations thereof. In yet another embodiment of the invention the composition does not comprise a physiologically active agent that is selected from drugs, diagnostic agents, essential oils, or physiologically active agents which are useful for cosmetic or nutritional purposes. Compositions comprising an above-described cellulose ether in combination with a tonicity-adjusting agent but not a physiologically active agent in addition are useful, e.g., for rinsing and/or moisturizing the nasal cavity or as artificial tears.

The composition for transmucosal delivery further comprises a liquid diluent, of which at least 55 weight percent and up to 100 percent is water. The composition of the present invention may additionally comprise an organic liquid diluent; however, the composition of the present invention should comprise at least 55, preferably at least 65, more preferably at least 75, most preferably at least 90, and particularly at least 95 weight percent of water and up to 45, preferably up to 35, more preferably up to 25, most preferably only up to 10, and particularly only up to 5 weight percent of an organic liquid diluent, based on the total weight of the organic liquid diluent and water. In one embodiment the diluent consists of water. The water is typically a high-quality grade of water such as purified water, for example USP purified water, PhEur purified water or water for Injection (WFI).

The term "organic liquid diluent" as used herein means an organic solvent or a mixture of two or more organic solvents that is liquid at 25 °C and atmospheric pressure. Preferred organic liquid diluents are polar organic solvents having one or more heteroatoms, such as oxygen, nitrogen or halogen (like chlorine). More preferred organic liquid diluents are alcohols, for example multifunctional alcohols, such as propylene glycol, polyethylene glycol, polypropylene glycol and glycerol; or preferably monofunctional alcohols, such as ethanol, isopropanol or n-propanol; or acetates, such as ethyl acetate. More preferably the organic liquid diluents have 1 to 6, most preferably 1 to 4 carbon atoms. The organic liquid diluent is preferably pharmaceutically acceptable, such as ethanol or glycerol.

The composition of the present invention may comprise one or more optional adjuvants, such as one or more suspending agents, odor, flavor or taste improvers, preservatives, pharmaceutically acceptable surfactants, coloring agents, opacifiers, or antioxidants. Typically, pharmaceutically acceptable optional adjuvants are selected.

For stability purposes, compositions of the invention (for example intranasal compositions) may be protected from microbial or fungal contamination and growth by inclusion of one or more preservatives. Examples of pharmaceutically acceptable anti-microbial agents or preservatives may include, but are not limited to, quaternary ammonium compounds (e.g. benzalkonium chloride, benzethonium chloride, cetrimide, cetylpyridinium chloride, lauralkonium chloride and myristyl picolinium chloride), mercurial agents (e.g. phenylmercuric nitrate, phenylmercuric acetate and thimerosal), alcoholic agents (e.g. chlorobutanol, phenylethyl alcohol and benzyl alcohol), antibacterial esters (e.g. esters of para-hydroxybenzoic acid), chelating agents such as disodium edetate (EDTA) and other anti-microbial agents such as chlorhexidine, chlorocresol, sorbic acid and its salts (such as potassium sorbate) and polymyxin. Examples of pharmaceutically acceptable anti-fungal agents or preservatives may include, but are not limited to, sodium benzoate, sorbic acid, sodium propionate, methylparaben, ethylparaben, propylparaben and butylparaben. The preservative(s), if included, are typically present in an amount of from 0.001 to 1%, such as from 0.015% to 0.5%, based on the total weight of the composition. In one embodiment, the preservative is selected from benzalkonium chloride, EDTA and/or potassium sorbate. In a further embodiment, the preservative is EDTA and/or potassium sorbate.

The composition of the present invention comprises from 0.1 to 6 percent, preferably from 0.2 to 5 percent, even more preferably from 0.5 to 4 percent, and most preferably from 0.5 to 3.5 percent of the cellulose ether defined above; typically from 0.1 to 10 percent, preferably from 0.2 to 8.0 percent, more preferably from 0.3 to 6.0 percent, even more preferably from 0.5 to 4.0 percent, and most preferably from 0.5 to 2.0 percent of a tonicity-adjusting agent; typically from 0 to 20 percent, or from 0.01 to 10 percent, or from 0.1 to 5 percent of a physiologically active agent, and typically from 0 to 30 percent, or from 0.01 to 20 percent, or from 0.1 to 10 percent, of one or more optional adjuvants, based on the total weight of the composition, the remainder being a liquid diluent. At least 55 percent, preferably at least 65 percent, more preferably at least 75 percent, most preferably at least 90 percent, and particularly at least 95 percent and up to 100 percent of the weight of the liquid diluent is water. The composition generally comprises a sufficient amount of liquid diluent that the composition is liquid at 5 °C. Preferably, the amount of the liquid diluent is at least 50 percent, more preferably at least 80 percent, and most preferably at least 90 percent, based on the total weight of the composition. The composition of the present invention preferably has a viscosity of from 1.2 to 10,000 mPa·s, more preferably from 2.4 to 8000 mPa·s, even more preferably from 10 to 5000 mPa·s, and most preferably from 20 to 1000 mPa·s, measured at 5 °C at a shear rate of 10 s⁻¹.

Preferably the composition of the present invention is a combination of the preferred embodiments described herein. e.g., the composition of the present invention preferably comprises a combination of i) a preferred tonicity-adjusting agent, ii) a preferred liquid diluent, iii) a preferred cellulose ether, which is defined by a combination of preferred hydroxyalkyl groups with a preferred MS(hydroxy)alkyl, a preferred DS(methyl), a preferred s23/s26 ratio and a preferred viscosity, and optionally iv) a preferred physiologically active agent and v) one or more preferred optional adjuvants, the components i), ii), iii), and optionally iv) and v) being comprised in preferred weight ranges in the composition, as described herein.

The composition of the present invention is preferably in the form of a sprayable solution or suspension, a solution or suspension to be applied as drops or in the form of a syrup. The composition can be packaged into suitable containers, which can also serve as delivery devices, e.g., containers designed for generating and subsequently applying sprays or drops to the intended delivery site. The delivery devices can be multi-dose or unit-dose devices.

The composition of the present invention is preferably packaged in a container such that the volume of the composition in the container is not more than 100 ml, more preferably not more than 50 ml or not more than 25 ml. Typically the volume of the composition in the container is at least 0.1 ml, or at least 1 ml, or at least 2 ml, or at least 5 ml, or at least 10 ml. The volume of the liquid composition typically depends on the intended use. Single-dose vials often comprise only 0.1 - 2 ml of a liquid composition. Sprays or bottles which are designed to release the composition drop by drop usually comprise larger amounts of the liquid composition, e.g., 5 - 50 ml or 10 - 25 ml.

The composition of the present invention is preferably stored at a temperature of from 1 to 23 °C, more preferably from 5 to 20 °C. Upon application of the composition of the present invention to a mucosa of an individual, the temperature of the composition increases and the cellulose ether suspended or, preferably, dissolved in the aqueous diluent of the composition precipitates or gels when the temperature of the composition of the present invention adjusts to the temperature of the mucosa, i.e., to a temperature of 30 - 37 °C, typically 30 - 35°C. The exact temperature of the mucosa somewhat depends on the type of mucosa, on the individual, on the time of day, and on the conditions of the surrounding environment. In the case of human beings the mucosa in the nasal cavity typically has a temperature of 30 - 35°C, the oral mucosa under the tongue typically has a temperature of about 36.8 ± 0.4°C, and the rectal mucosa typically has a temperature of about 37°C.

The gelation of the composition can be determined as described in the Examples section. Preferred embodiments of the composition of the present invention are particularly useful for application to the nasal mucosa. Generally, the composition of the present invention exhibits a gelation temperature of up to 37 °C, in a preferred embodiment up to 35 °C, and in another preferred embodiment up to 33 °C.

The gelation behavior of the cellulose ether utilized in the compositions of the present invention can be adapted to a certain degree to the specific need of a certain transmucosal delivery system. E.g., the desired viscosity increase of the composition of the present invention can be achieved at a lower temperature when the composition has a higher concentration of the cellulose ether than when its concentration is lower. Furthermore, a cellulose ether utilized in the compositions of the present invention that has a MS(hydroxyalkyl) of 0.05 to 0.25 or 0.05 to 0.20 or even 0.05 to 0.15 generally gels at a lower temperature in the compositions of the present invention than a comparable cellulose ether that has a higher MS(hydroxyalkyl).

Some embodiments of the invention will now be described in detail in the following Examples.

### EXAMPLES

Unless otherwise mentioned, all parts and percentages are by weight. In the Examples the following test procedures are used.

### Determination of the DS(methyl) and the MS(hydroxypropyl) of hydroxypropyl methylcellulose (HPMC)

The determination of the % methoxyl and % hydroxypropoxyl was carried out according to the United States Pharmacopeia (USP 35, "Hypromellose", pages 3467-3469). The values obtained are % methoxyl and % hydroxypropoxyl. These are subsequently converted into degree of substitution (DS) for methoxyl substituents and molar substitution (MS) for hydroxypropoxyl substituents. Residual amounts of salt have been taken into account in the conversion.

### Production of a 2 % pure aqueous solution of the HPMC

To obtain a 2 % aqueous solution of HPMC, 3 g of milled, ground, and dried HPMC (under consideration of the water content of the HPMC) were added to 147 g of tap water (temperature 20 - 25 °C) at room temperature while stirring with an overhead lab stirrer at 750 rpm with 3-wing (wing = 2 cm) blade stirrer. The solution was then cooled to about 5 °C. After the temperature of 5 °C was reached the solution was stirred for 5 h at 750 rpm and stored in a refrigerator overnight. Prior to use or analysis, the solution was stirred for 15 min at 100 rpm in an ice bath.

### Determination of the viscosity of HPMC

The steady-shear-flow viscosity η (20 °C, 10 s⁻¹, 2 wt.% HPMC) of an aqueous 2 wt.% HPMC solution was measured at 20 °C at a shear rate of 10 s⁻¹ with an Anton Paar Physica MCR 501 rheometer and cup and bob fixtures (CC-27).

### Determination of s23/s26 of HPMC

The determination of ether substituents in cellulose ethers is generally known and e.g., described in Carbohydrate Research, 176 (1988) 137-144, Elsevier Science Publishers B.V., Amsterdam, DISTRIBUTION OF SUBSTITUENTS IN O-ETHYL-O-(2-HYDROXYETHYL)CELLULOSE by Bengt Lindberg, Ulf Lindquist, and
Olle Stenberg.

Specifically, determination of s23/s26 is conducted as follows:
10-12 mg of the cellulose ether are dissolved in 4.0 mL of dry analytical grade dimethyl sulfoxide (DMSO) (Merck, Darmstadt, Germany, stored over 0.3nm molecular sieve beads) at about 90 °C under stirring and then cooled down to room temperature again. The solution is left stirring at room temperature over night to ensure complete solubilization. The entire reaction including the solubilization of the cellulose ether is performed using a dry nitrogen atmosphere in a 4 mL screw cap vial. After solubilization the dissolved cellulose ether is transferred to a 22 mL screw cap vial. Powdered sodium hydroxide (freshly pestled, analytical grade, Merck, Darmstadt, Germany) and ethyl iodide (for synthesis, stabilized with silver, Merck-Schuchardt, Hohenbrunn, Germany) in a thirty fold molar excess of the reagents sodium hydroxide and ethyl iodide per hydroxyl group of the anhydroglucose unit are added and the solution is vigorously stirred under nitrogen in the dark for three days at ambient temperature. The perethylation is repeated with addition of the threefold amount of the reagents sodium hydroxide and ethyl iodide compared to the first reagent addition and further stirring at room temperature for additional two days. Optionally the reaction mixture can be diluted with up to 1.5 mL DMSO to ensure good mixing during the course of the reaction. 5 mL of 5 % aqueous sodium thiosulfate solution is poured into the reaction mixture and the obtained solution is then extracted three times with 4 mL of dichloromethane. The combined extracts are washed three times with 2 mL of water. The organic phase is dried with anhydrous sodium sulfate (ca. 1g). After filtration the solvent is removed in a gentle stream of nitrogen and the sample is stored at 4 °C until further sample preparation.

Hydrolysis of about 5 mg of the perethylated samples is performed under nitrogen in a 2 mL screw cap vial with 1 mL of 90 % aqueous formic acid under stirring at 100 °C for 1 hour. The acid is removed in a stream of nitrogen at 35-40 °C and the hydrolysis is repeated with 1 mL of 2M aqueous trifluoroacetic acid for 3 hours at 120 °C in an inert nitrogen atmosphere under stirring. After completion the acid is removed to dryness in a stream of nitrogen at ambient temperature using ca. 1 mL of toluene for co-distillation.

The residues of the hydrolysis are reduced with 0.5 mL of 0.5 M sodium borodeuteride in 2N aqueous ammonia solution (freshly prepared) for 3 hours at room temperature under stirring. The excess reagent is destroyed by drop wise addition of ca. 200 µL of concentrated acetic acid. The resulting solution is evaporated to dryness in a stream of nitrogen at ca. 35-40 °C and subsequently dried in vacuum for 15 min at room temperature. The viscous residue is dissolved in 0.5 mL of 15 % acetic acid in methanol and evaporated to dryness at room temperature. This is done five times and repeated four times with pure methanol. After the final evaporation the sample is dried in vacuum overnight at room temperature.

The residue of the reduction is acetylated with 600 µL of acetic anhydride and 150 µL of pyridine for 3 hrs at 90 °C. After cooling the sample vial is filled with toluene and evaporated to dryness in a stream of nitrogen at room temperature. The residue is dissolved in 4 mL of dichloromethane and poured into 2 mL of water and extracted with 2 mL of dichloromethane. The extraction is repeated three times. The combined extracts are washed three times with 4 mL of water and dried with anhydrous sodium sulfate. The dried dichloromethane extract is subsequently submitted to GC analysis. Depending on the sensitivity of the GC system, a further dilution of the extract can be necessary.

Gas-liquid (GLC) chromatographic analyses are performed with Hewlett Packard 5890A and 5890A Series II type of gas chromatographs equipped with J&W capillary columns DB5, 30 m, 0.25 mm ID, 0.25 µm phase layer thickness operated with 1.5 bar helium carrier gas. The gas chromatograph is programmed with a temperature profile that holds constant at 60 °C for 1 min, heats up at a rate of 20 °C / min to 200 °C, heats further up with a rate of 4 °C / min to 250 °C, heats further up with a rate of 20 °C / min to 310 °C where it is held constant for another 10 min. The injector temperature is set to 280 °C and the temperature of the flame ionization detector (FID) is set to 300 °C. 1µL of the samples is injected in the splitless mode at 0.5 min valve time. Data are acquired and processed with a LabSystems Atlas work station.

Quantitative monomer composition data are obtained from the peak areas measured by GLC with FID detection. Molar responses of the monomers are calculated in line with the effective carbon number (ECN) concept but modified as described in the table below. The effective carbon number (ECN) concept has been described by Ackman (R.G. Ackman, J. Gas Chromatogr., 2 (1964) 173-179 and R.F. Addison, R.G. Ackman, J. Gas Chromatogr., 6 (1968) 135-138) and applied to the quantitative analysis of partially alkylated alditol acetates by Sweet et. al (D.P. Sweet, R.H. Shapiro, P. Albersheim, Carbohyd. Res., 40 (1975) 217-225).

### ECN increments used for ECN calculations:

| Type of carbon atom | ECN increment |
|---|---|
| hydrocarbon | 100 |
| primary alcohol | 55 |
| secondary alcohol | 45 |

In order to correct for the different molar responses of the monomers, the peak areas are multiplied by molar response factors MRFmonomer which are defined as the response relative to the 2,3,6-Me monomer. The 2,3,6-Me monomer is chosen as reference since it is present in all samples analyzed in the determination of s23 / s26.
MRFmonomer = ECN2,3,6-Me / ECNmonomer

The mole fractions of the monomers are calculated by dividing the corrected peak areas by the total corrected peak area according to the following formulas:
s23 = [ (23-Me + 23-Me-6-HAMe + 23-Me-6-HA + 23-Me-6-HAHAMe + 23-Me-6-HAHA ]; and
s26 = [ (26-Me + 26-Me-3-HAMe + 26-Me-3-HA + 26-Me-3-HAHAMe + 26-Me-3-HAHA], wherein
s23 is the sum of the molar fractions of anhydroglucose units which meet the following conditions:
a) the two hydroxy groups in the 2- and 3-positions of the anhydroglucose unit are substituted with methyl groups and the 6-position is not substituted (= 23-Me);
b) the two hydroxy groups in the 2- and 3-positions of the anhydroglucose unit are substituted with methyl groups and the 6-position is substituted with methylated hydroxyalkyl (= 23-Me-6-HAMe) or with a methylated side chain comprising 2 hydroxyalkyl groups (= 23-Me-6-HAHAMe); and
c) the two hydroxy groups in the 2- and 3-positions of the anhydroglucose unit are substituted with methyl groups and the 6-position is substituted with hydroxyalkyl (= 23-Me-6-HA) or with a side chain comprising 2 hydroxyalkyl groups (= 23-Me-6-HAHA).
s26 is the sum of the molar fractions of anhydroglucose units which meet the following conditions:
a) the two hydroxy groups in the 2- and 6-positions of the anhydroglucose unit are substituted with methyl groups and the 3-position is not substituted (= 26-Me);
b) the two hydroxy groups in the 2- and 6-positions of the anhydroglucose unit are substituted with methyl groups and the 3-position is substituted with methylated hydroxyalkyl (= 26-Me-3-HAMe) or with a methylated side chain comprising 2 hydroxyalkyl groups (= 26-Me-3-HAHAMe); and
c) the two hydroxy groups in the 2- and 6-positions of the anhydroglucose unit are substituted with methyl groups and the 3-position is substituted with hydroxyalkyl (= 26-Me-3-HA) or with a side chain comprising 2 hydroxyalkyl groups (= 26-Me-3-HAHA).

The results of the determination of the substituents in the HAMC are listed in Table 3 below. In the case of HPMC's hydroxyalkyl (HA) is hydroxypropyl (HP) and methylated hydroxyalkyl (HAMe) is methylated hydroxypropyl (HPMe).

### Production of HPMC-A

Hydroxypropyl methylcellulose (HPMC) is produced according to the following procedure. Finely ground wood cellulose pulp is loaded into a jacketed, agitated reactor. The reactor is evacuated and purged with nitrogen to remove oxygen and then evacuated again. The reaction is carried out in two stages. In the first stage a 50 weight percent aqueous solution of sodium hydroxide is sprayed onto the cellulose in an amount of 2.0 moles of sodium hydroxide per mole of anhydroglucose units in the cellulose and the temperature is adjusted to 40°C. After stirring the mixture of aqueous sodium hydroxide solution and cellulose for about 20 minutes at 40°C, 1.5 moles of dimethyl ether, 2.5 moles of methyl chloride and 0.2 mols of propylene oxide per mole of anhydroglucose units are added to the reactor. The contents of the reactor are then heated in 60 min to 80°C. After having reached 80°C, the first stage reaction is allowed to proceed for 30 min.

The second stage of the reaction is started by addition of methyl chloride in an amount of 2.8 molar equivalents of methyl chloride per mole of anhydroglucose units. The addition time for methyl chloride is 10 min. Then a 50 weight percent aqueous solution of sodium hydroxide at an amount of 2.3 moles of sodium hydroxide per mole of anhydroglucose units is added over a time period of 90 min. The rate of addition is 0.026 moles of sodium hydroxide per mole of anhydroglucose units per minute. After the second stage addition is completed the contents of the reactor are then kept at a temperature of 80 °C for 120 min.

After the reaction, the reactor is vented and cooled down to about 50°C. The contents of the reactor are removed and transferred to a tank containing hot water. The crude HPMC is then neutralized with formic acid and washed chloride free with hot water (assessed by AgNO₃ flocculation test), cooled to room temperature and dried at 55 °C in an air-swept drier. The material is then ground using an Alpine UPZ mill using a 0.5 mm screen.

Production of HPMC-B Example 1 is repeated, except that the amount of propylene oxide added to the reaction mixture is 0.4 mols of propylene oxide per mole of anhydroglucose units.

### Production of HPMC-C

Example 1 is repeated, except that the amount of propylene oxide added to the reaction mixture is 0.6 mols of propylene oxide per mole of anhydroglucose units.

### Production of HPMC-1, HPMC-2, HPMC-3 and HPMC-4

HPMC-A, HPMC-B and HPMC-C are partially depolymerized by heating the powderous samples with gaseous hydrogen chloride at a time and temperature listed in Table 1 below. The partially depolymerized hydroxypropyl methylcellulose is neutralized with sodium bicarbonate.

**Table 1**

| **HPMC type** | **HPMC-1** | **HPMC-2** | **HPMC-3** | **HPMC-4** |
|---|---|---|---|---|
| Feedstock material | HPMC-A | HPMC-B | HPMC-C | HPMC-B |
| Weight in g of HCl, based on weight in kg of feedstock material | 1.8 | 1.8 | 2.6 | 1.5 |
| degradation time [min] | 120 | 120 | 120 | 60 |
| Degradation temp. [°C] | 85 | 85 | 85 | 75 |
| Viscosity as 2 wt.% aqueous solution at 20°C [m·Pas] | 9.2 | 5.8 | 4.8 | 216 |

| | | | | |
|---|---|---|---|---|
| METHOCEL™ E4M, METHOCEL™ F4M, METHOCEL™ K4M, METHOCEL™ F5 and METHOCEL™ E5 cellulose ethers METHOCEL™ E4M, METHOCEL™ F4M, METHOCEL™ K4M, METHOCEL™ F5 and METHOCEL™ E5 cellulose ethers are commercially available from The Dow Chemical Company and abbreviated as E4M, F4M, K4M, F5 and E5. | | | | |

### Comparative Examples C-20 and C-21

METHOCEL™ F4M was partially depolymerized by contacting it with 1.5 g gaseous HCl per g of hydroxypropyl methylcellulose at a temperature of 70°C for 50 min. The METHOCEL™ F4M used for partial depolymerization originated from a different batch than the one abbreviated as F4M in Table 2 below and had a slightly different DS and MS. Subsequently the HCl gas was removed by evacuation. The hydroxypropyl methylcellulose was cooled to room temperature and subsequently neutralized with sodium bicarbonate.
The properties of the HPMCs are listed in Table 2 below.

**Table 2**

| **HPMC type** | **Abbreviation** | **DS (methyl)** | **MS (hydroxypropyl)** | **2% Viscosity at 10 s⁻¹ at 20°C, [mPa·s]** | **s23/s26** |
|---|---|---|---|---|---|
| HPMC-A | HPMC-A | 1.80 | 0.08 | 7160 | 0.19 |
| HPMC-1 | HPMC-1 | 1.80 | 0.08 | 9.2 | 0.19 |
| HPMC-B | HPMC-B | 1.81 | 0.15 | 8770 | 0.22 |
| HPMC-2 | HPMC-2 | 1.81 | 0.15 | 5.8 | 0.22 |
| HPMC-4 | HPMC-4 | 1.81 | 0.15 | 216 | 0.22 |
| HPMC-C | HPMC-C | 1.82 | 0.20 | 9050 | 0.22 |
| HPMC-3 | HPMC-3 | 1.82 | 0.20 | 4.8 | 0.22 |
| METHOCEL™ F5 | F5 | 1.90 | 0.16 | 4.6 | 0.42 |
| METHOCEL™ E5 | E5 | 1.89 | 0.25 | 5.6 | 0.38 |
| METHOCEL™ E4M | E4M | 1.93 | 0.23 | 5045 | 0.39 |
| METHOCEL™ F4M | F4M | 1.88 | 0.15 | 4970 | 0.42 |
| METHOCEL™ K4M | K4M | 1.45 | 0.22 | 5494 | 0.45 |
| Partially depolymerized METHOCEL™ F4M | F250 | 1.87 | 0.16 | 248 | 0.43 |

**Table 3**

| **HPMC type** | **HPMC-A / HPMC-1** | **HPMC-B / HPMC-2 / HPMC-4** | **HPMC-C / HPMC-3** | **METHOCEL F5** | **METHOCEL E5** | **METHOCEL E4M** | **METHOCEL F4M** | **METHOCEL K4M** | **F250** |
|---|---|---|---|---|---|---|---|---|---|
| DS (USP) | 1.8 | 1.81 | 1.82 | 1.90 | 1.89 | 1.93 | 1.88 | 1.45 | 1.87 |
| MS (USP) | 0.08 | 0.15 | 0.2 | 0.16 | 0.25 | 0.23 | 0.15 | 0.22 | 0.16 |
| mol fraction (26-Me) | 0.3215 | 0.2984 | 0.2861 | 0.2329 | 0.2193 | 0.2207 | 0.2306 | 0.1885 | 0.2281 |
| mol fraction (26-Me-3-HA) | 0.0056 | 0.0124 | 0.0161 | 0.0131 | 0.0207 | 0.0211 | 0.0138 | 0.0138 | 0.0143 |
| mol fraction (26-Me-3-HAHA) | 0.0000 | 0.0000 | 0.0000 | 0.0000 | 0.0000 | 0.0000 | 0.0000 | 0.0000 | 0.0000 |
| mol fraction (26-Me-3HAMe) | 0.0011 | 0.0019 | 0.0022 | 0.0027 | 0.0051 | 0.0047 | 0.0029 | 0.0020 | 0.0024 |
| mol fraction (26-Me-3HAHAMe) | 0.0000 | 0.0000 | 0.0000 | 0.0000 | 0.0000 | 0.0000 | 0.0000 | 0.0000 | 0.0000 |
| mol fraction (23-Me) | 0.0618 | 0.0600 | 0.0572 | 0.0966 | 0.0799 | 0.0835 | 0.0951 | 0.0822 | 0.0959 |
| mol fraction (23-Me-6-HA) | 0.0019 | 0.0074 | 0.0091 | 0.0086 | 0.0134 | 0.0134 | 0.0091 | 0.0100 | 0.0100 |
| mol fraction (23-Me-6-HAHA) | 0.0000 | 0.0000 | 0.0000 | 0.0000 | 0.0000 | 0.0000 | 0.0000 | 0.0000 | 0.0000 |
| mol fraction (23-Me-6-HAMe) | 0.0000 | 0.0000 | 0.0000 | 0.0000 | 0.0000 | 0.0000 | 0.0000 | 0.0000 | 0.0000 |
| mol fraction (23-Me-6-HAHAMe) | 0.0000 | 0.0000 | 0.0000 | 0.0000 | 0.0000 | 0.0000 | 0.0000 | 0.0000 | 0.0000 |
| s23/s26 | 0.19 | 0.22 | 0.22 | 0.42 | 0.38 | 0.39 | 0.42 | 0.45 | 0.43 |

### Preparation of aqueous solutions for application to the nasal mucosa

Concentrated HPMC solutions having the concentrations as listed in Table 4 below were prepared by adding a corresponding amount of dry HPMC powder to water which had an initial temperature of >80 °C using a magnetic stir bar to achieve a good dispersion. The mixture of the HPMC and water was cooled to 5 °C within 20 minutes while stirring at the same speed. After the mixture of HPMC and water reached the temperature of 5 °C, the mixture was stirred for one additional hour at this temperature. These solutions were stored overnight in a refrigerator.

**Table 4**

| **HPMC type** | **MC concentration in concentrated MC solution [wt.-%]** |
|---|---|
| HPMC-1, HPMC-2 or HPMC-3, METHOCEL™ F5 or METHOCEL™ E5 | 10 |
| HPMC-4, partially depolymerized METHOCEL™ F4M | 4 |
| HPMC-A, HPMC-B, METHOCEL™ E4M, METHOCEL™ F4M or METHOCEL™ K4M | 2 |

A concentrated HPMC solution from the refrigerator was used as such if appropriate or mixed either with water or with a concentrated aqueous solution of a tonicity-adjusting agent and/or an active pharmaceutical ingredient (API) from the refrigerator by the use of a magnetic stirring bar until a homogenous solution was achieved. Acetaminophen was used as API. During this additional mixing the solution temperature was still cold and a temperature above 7 °C was not reached. The concentration of the tonicity-adjusting agent and/or the API in the concentrated aqueous solution was chosen to achieve the desired concentration in the resulting mixture at the chosen mixing ratio. In the resulting mixture the tonicity-adjusting agent was the salt in a pH 6.5 phosphate buffered solution (PBS) which comprised 0.65 weight percent sodium chloride and 0.25 weight percent sodium phosphate in deionized water. For example, the aqueous solution of Example I-1 was prepared by mixing equal parts of a solution containing 10 wt. % HPMC-3 with a solution containing 1.3 wt. % sodium chloride and 0.5 wt. % sodium phosphate to generate the 5 wt. % HPMC solution in phosphate buffered saline (0.65% sodium chloride, 0.25% sodium phosphate). The aqueous solutions of all Examples and Comparative Examples were fully soluble, clear solutions and were stored in the refrigerator until the characterization had been performed.

### Determination of the viscosity of aqueous compositions for application to a mucosa comprising HPMC

The viscosities of aqueous compositions for application to a mucosa were measured using an ARES RFS3 rheometer with cup and bob fixtures (TA-Instruments) at 5 °C and at a shear rate of 10 s⁻¹.

### Determining the gelation temperature of aqueous compositions comprising HPMC

Rheology measurements of aqueous solutions comprising HPMC and optionally a tonicity-adjusting agent, such as a buffering agent, and/or a physiologically active agent were conducted with an Ares RFS3 rheometer (TA-Instruments) with cup and bob fixtures. Seventeen milliliters of solution were transferred to the cup fixture and the gap set to 5 mm. The sample was heated at a rate of 1°C per minute over a temperature range from 10 to 50 °C with a constant strain of 2% and a constant angular frequency of 5 radians per second.

The storage modulus G', which is obtained from the oscillation measurements, represents the elastic properties of the solution. The loss modulus G", which is obtained from the oscillation measurements, represents the viscous properties of the solution. At low temperature the loss modulus values G" are higher than the storage modulus G' and both values are slightly decreasing with increasing temperatures. With further increasing temperatures the storage modulus values are increasing and a cross-over between the storage modulus and the loss modulus is obtained. The cross-over of G' and G" is determined to be the gelation temperature. Some HPMCs might show two points of cross-over of G' and G". In such case the gelation temperature is the temperature at which G'/G" = 1 and G" > G' at a temperature which is 1 °C colder than G'/G" = 1.

The chemical compositions of the aqueous solutions, their gelation temperatures and their viscosities are listed in Table 5 below.

**Table 5**

| | **HPMC properties** | | | | | **Properties of Composition** | | | | |
|---|---|---|---|---|---|---|---|---|---|---|
| **(Comp.) Example** | **HPMC** | **DS of HPMC** | **MS of HPMC** | **HMPC viscosity, 2 wt.% in water at 20 °C,10s⁻¹, mPa·s** | **s23/s26 of HPMC** | **HPMC conc., wt.-%** | **liquid** | **API, wt.-%** | **Gelation Temperature, °C** | **Solution viscosity at 5 °C**, **10 s⁻¹, mPa•s** |
| C-1 | HPMC-3 | 1.82 | 0.20 | 4.8 | 0.22 | 5 | water | -- | 40 | 49 |
| I-1 | HPMC-3 | 1.82 | 0.20 | 4.8 | 0.22 | 5 | PBS | -- | 36 | 57 |
| I-2 | HPMC-3 | 1.82 | 0.20 | 4.8 | 0.22 | 5 | PBS | 1 | 35 | 53 |
| C-2 | HPMC-3 | 1.82 | 0.20 | 4.8 | 0.22 | 10 | water | -- | 31 | Nm |
| C-3 | HPMC-2 | 1.81 | 0.15 | 5.8 | 0.22 | 5 | water | -- | 35 | Nm |
| I-3 | HPMC-2 | 1.81 | 0.15 | 5.8 | 0.22 | 5 | PBS | -- | 31 | Nm |
| C-4 | HPMC-2 | 1.81 | 0.15 | 5.8 | 0.22 | 3 | water | -- | 38 | 21 |
| I-4 | HPMC-2 | 1.81 | 0.15 | 5.8 | 0.22 | 3 | PBS | -- | 34 | 24 |
| C-5 | HPMC-2 | 1.81 | 0.15 | 5.8 | 0.22 | 10 | water | -- | 27 | Nm |
| C-6 | HPMC-4 | 1.81 | 0.15 | 216 | 0.22 | 2 | water | -- | 41 | 408 |
| I-5 | HPMC-4 | 1.81 | 0.15 | 216 | 0.22 | 2 | PBS | -- | 37 | 430 |
| C-7 | HPMC-B | 1.81 | 0.15 | 8710 | 0.22 | 1.5 | water | -- | 46 | Nm |
| C-8 | HPMC-B | 1.81 | 0.15 | 8710 | 0.22 | 1.5 | PBS | -- | 40 | Nm |
| C-9 | HPMC-B | 1.81 | 0.15 | 8710 | 0.22 | 1.5 | 3% NaCl | -- | 35 | Nm |
| C-10 | HPMC-B | 1.81 | 0.15 | 8710 | 0.22 | 1.5 | PBS | 1 | 39 | Nm |
| C-11 | HPMC-B | 1.81 | 0.15 | 8710 | 0.22 | 1.5 | 3% NaCl | 1 | 33 | Nm |
| C-12 | HPMC-1 | 1.80 | 0.08 | 9.2 | 0.19 | 3 | water | -- | 31 | Nm |
| I-6 | HPMC-1 | 1.80 | 0.08 | 9.2 | 0.19 | 3 | PBS | -- | 28 | Nm |
| C-13 | HPMC-1 | 1.80 | 0.08 | 9.2 | 0.19 | 1.5 | water | -- | 38 | 15 |
| I-7 | HPMC-1 | 1.80 | 0.08 | 9.2 | 0.19 | 1.5 | PBS | -- | 33 | 12 |
| C-14 | HPMC-1 | 1.80 | 0.08 | 9.2 | 0.19 | 8 | water | -- | 26 | Nm |
| C-15 | HPMC-A | 1.80 | 0.08 | 6840 | 0.19 | 1.5 | water | -- | 40 | 6460 |
| I-8 | HPMC-A | 1.80 | 0.08 | 6840 | 0.19 | 1.5 | PBS | -- | 37 | 4910 |
| I-9 | HPMC-A | 1.80 | 0.08 | 6840 | 0.19 | 1.5 | 3% NaCl | -- | 29 | 5010 |
| C-16 | E4M | 1.93 | 0.23 | 5045 | 0.39 | 1.5 | 3% NaCl | -- | 46 | Nm |
| C-17 | F4M | 1.88 | 0.15 | 4970 | 0.42 | 1.5 | 3% NaCl | -- | 40 | Nm |
| C-18 | K4M | 1.45 | 0.22 | 5494 | 0.45 | 1.5 | 3% NaCl | -- | >45 | Nm |
| C-19 | F5 | 1.90 | 0.16 | 4.6 | 0.42 | 5 | 3% NaCl | 1 | 42 | Nm |
| C-20 | E5 | 1.89 | 0.25 | 5.6 | 0.38 | 5 | 3% NaCl | 1 | 40 | Nm |
| C-21 | F250 | 1.88 | 0.15 | 248 | 0.42 | 2 | water | -- | > 50 | 560 |
| C-22 | F250 | 1.88 | 0.15 | 248 | 0.42 | 2 | PBS | -- | > 50 | 565 |

| | | | | | | | | | | |
|---|---|---|---|---|---|---|---|---|---|---|
| Nm: not measured | | | | | | | | | | |

The comparison between Examples I-1 and I-2 on one hand and Comparative Example C-1 on the other hand in Table 5 above illustrates that a composition which comprises a hydroxyalkyl methylcellulose with an s23/s26 of 0.29 or less in combination with a tonicity-adjusting agent has a lower gelation temperature than a composition that comprises the same hydroxyalkyl methylcellulose alone. The composition of Comparative Example C-1 gels above the normal temperature of a human mucosa and, accordingly, does not have same benefits as the compositions of Examples I-1 and I-2.

The comparisons between Example I-3 and Comparative Example C-3, between Example I-4 and Comparative Example C-4, and between Example I-5 and Comparative Example C-6 respectively illustrate the same effect of a hydroxyalkyl methylcellulose with an s23/s26 of 0.29 or less in combination with a tonicity-adjusting agent as discussed above, i.e., lowering the gelation temperature of the composition. The comparison between Examples I-8 and I-9 on the one hand and Comparative Example C-15 on the other hand again illustrates the same effect.

Comparative Examples C-1 and C-2 illustrate that the gelation temperature of an aqueous composition depends on the concentration of the hydroxyalkyl methylcellulose. When the concentration of the hydroxyalkyl methylcellulose in the aqueous composition is increased, the gelation temperature decreases and vice versa. For economical reasons it is desirable to keep the concentration of the hydroxyalkyl methylcellulose as low as possible.

The comparisons between Examples I-4 and I-3, between Examples I-7 and I-6, between Comparative Examples C-4, C-3 and C-5 and between Comparative Examples C-13, C-12 and C-14 again illustrate that the gelation temperature decreases when the concentration of the hydroxyalkyl methylcellulose in the aqueous composition is increased, and vice versa. The reduced gelation temperature due to the presence of a tonicity-adjusting agent allows a reduction of the concentration of hydroxyalkyl methylcellulose.

Comparative Examples C-8 and C-10 illustrate that the use of hydroxyalkyl methylcellulose having a viscosity of more than 8000 mPa•s, measured as 2 wt. % aqueous solution at 20 °C at a shear rate of 10 s⁻¹ usually does not provide the desired benefits in a composition for application to a mucosa, even when the s23/s26 is 0.29 or less and even in the presence of a certain tonicity-adjusting agent. The comparisons between Comparative Examples C-8 and C-9 and between Comparative Examples C-10 and C-11 illustrate that the use of 3% NaCl as a tonicity-adjusting agent decreases the gelation temperature more than phosphate buffered solution (PBS) that comprises a lower amount of NaCl. However, the use of hydroxyalkyl methylcellulose having a viscosity of more than 8000 mPa•s is disadvantageous because it unduly limits the choice of tonicity-adjusting agents. Comparative Examples C-7 to C-11 are not prior art.

Comparative Examples C-16 to C-20 illustrate that hydroxyalkyl methylcellulose with an s23/s26 of more than 0.29 do not provide the above-mentioned benefits in compositions for application to a mucosa, even if they have a viscosity of up to 8000 mPa•s, measured as 2 wt. % aqueous solution at 20 °C at a shear rate of 10 s⁻¹, and even if they are used in combination with 3% NaCl as a tonicity-adjusting agent. These compositions do not gel at the temperature of a mucosa.

Comparative Examples C-21 and C-22 illustrate that the hydroxyalkyl methylcellulose with an s23/s26 of more than 0.29 gels above the normal temperature of a human mucosa, even if it is used in combination with a tonicity-adjusting agent. The hydroxyalkyl methylcellulose in the solutions of Comparative Examples C-21 and C-22 gels at a temperature that is significantly higher than that of HPMC-4, which has an s23/s26 of 0.29 or less, in the comparable solutions of C-6 and I-5.

## Claims

1. A composition for application to a mucosa comprising
i) from 0.1 to 10 weight percent of a tonicity-adjusting agent, based on the total weight of the composition,
ii) a liquid diluent of which at least 55 weight percent is water, and
iii) from 0.1 to 6 weight percent of a cellulose ether, based on the total weight of the composition,
wherein the cellulose ether has a viscosity of from 1.2 to 8000 mPa•s, measured as 2 wt. % aqueous solution at 20 °C at a shear rate of 10 s⁻¹, and
wherein the cellulose ether has anhydroglucose units joined by 1-4 linkages and has methyl groups, hydroxyalkyl groups, and optionally alkyl groups being different from methyl as substituents such that
hydroxyl groups of anhydroglucose units are substituted with methyl groups such that s23/s26 is 0.29 or less,
wherein s23 is the molar fraction of anhydroglucose units wherein only the two hydroxyl groups in the 2- and 3-positions of the anhydroglucose unit are substituted with a methyl group and
wherein s26 is the molar fraction of anhydroglucose units wherein only the two hydroxyl groups in the 2- and 6-positions of the anhydroglucose unit are substituted with a methyl group.

2. The composition of claim 1 wherein the tonicity-adjusting agent is an alkali or alkaline earth metal halide, dextrose, xylitol, glucose, mannitol, or sorbitol.

3. The composition of claim 1 or 2 additionally comprising a physiologically active agent.

4. A composition for use as a medicament comprising a physiologically active agent selected from one or more drugs or one or more diagnostic agents and
i) from 0.1 to 10 weight percent of a tonicity-adjusting agent, based on the total weight of the composition,
ii) a liquid diluent of which at least 55 weight percent is water, and
iii) from 0.1 to 6 weight percent of a cellulose ether, based on the total weight of the composition,
wherein the cellulose ether has a viscosity of from 1.2 to 8000 mPa•s, measured as 2 wt. % aqueous solution at 20 °C at a shear rate of 10 s⁻¹, and
wherein the cellulose ether has anhydroglucose units joined by 1-4 linkages and has methyl groups, hydroxyalkyl groups, and optionally alkyl groups being different from methyl as substituents such that
hydroxyl groups of anhydroglucose units are substituted with methyl groups such that s23/s26 is 0.29 or less,
wherein s23 is the molar fraction of anhydroglucose units wherein only the two hydroxyl groups in the 2- and 3-positions of the anhydroglucose unit are substituted with a methyl group and
wherein s26 is the molar fraction of anhydroglucose units wherein only the two hydroxyl groups in the 2- and 6-positions of the anhydroglucose unit are substituted with a methyl group.

5. The composition of any one of claims 1 to 4 wherein the cellulose ether has a viscosity from 1.8 to 6000 mPa•s, measured as 2 wt. % aqueous solution at 20 °C at a shear rate of 10 s⁻¹.

6. The composition of any one of claims 1 to 5 wherein the cellulose ether has a viscosity of from 2.4 to 1000 mPa•s, measured as 2 wt. % aqueous solution at 20 °C at a shear rate of 10 s⁻¹.

7. The composition of any one of claims 1 to 6 wherein the cellulose ether has an MS(hydroxyalkyl) of 0.05 to 0.35.

8. The composition of any one of claims 1 to 7 comprising from 0.2 to 8.0 weight percent of a tonicity-adjusting agent, based on the total weight of the composition.

9. The composition of any one of claims 1 to 8 having a viscosity of from 2.4 to 8000 mPa•s, measured at 5 °C and at a shear rate of 10 s⁻¹.

10. The composition of any one of claims 1 to 9 exhibiting a gelation temperature of from 18 to 37 °C.

11. The composition of any one of claims 1 to 10 for intranasal administration.

12. The composition of any one of claims 1 to 11 comprising from 0.1 to 6 percent of the cellulose ether, from 0.1 to 10 percent of a tonicity-adjusting agent, from 0 to 20 percent of a physiologically active agent, and from 0 to 30 percent of one or more optional adjuvants, based on the total weight of the composition, the remainder being the liquid diluent.

13. The composition of any one of claims 3 to 12 wherein the physiologically active agent is selected from one or more drugs, one or more diagnostic agents, one or more essential oils, or one or more physiologically active agents which are useful for cosmetic or nutritional purposes.

14. A container comprising the composition of any one of claims 1 to 13, wherein the container is designed to release the composition by spraying or as drops.

15. The composition of any of claims 3 to 13 for the use in transmucosal administration of a physiologically active agent to an individual wherein said composition is applied to a mucosa of the individual.

## Patentansprüche

1. Zusammensetzung zur Aufbringung auf eine Schleimhaut, enthaltend
i) 0,1 bis 10 Gew.-% eines die Tonizität einstellenden Mittels, bezogen auf das Gesamtgewicht der Zusammensetzung,
ii) ein flüssiges Verdünnungsmittel, von dem wenigstens 55 Gew.-% Wasser sind, und
iii) 0,1 bis 6 Gew.-% eines Celluloseethers, bezogen auf das Gesamtgewicht der Zusammensetzung,
wobei der Celluloseether eine Viskosität von 1,2 bis 8000 mPa·s, gemessen als eine 2-gew.-%ige wässrige Lösung bei 20°C und einer Scherrate von 10 s⁻¹, aufweist und
wobei der Celluloseether Anhydroglucoseeinheiten aufweist, die über 1-4-Verknüpfungen miteinander verbunden sind, und Methylgruppen, Hydroxyalkylgruppen und optional Alkylgruppen, die sich von Methyl unterscheiden, als Substituenten aufweist, so dass
die Hydroxylgruppen der Anhydroglucoseeinheiten mit Methylgruppen substituiert sind, so dass s23/s26 gleich 0,29 oder kleiner ist,
wobei s23 der Molenbruch der Anhydroglucoseeinheiten ist, bei denen nur die zwei Hydroxylgruppen in den 2- und 3-Positionen der Anhydroglucoseeinheiten mit einer Methylgruppe substituiert sind, und
worin s26 der Molenbruch der Anhydroglucoseeinheiten ist, bei denen nur die zwei Hydroxylgruppen in den 2- oder 6-Positionen der Anhydroglucoseeinheit mit einer Methylgruppe substituiert sind.

2. Zusammensetzung nach Anspruch 1, wobei das Mittel zur Einstellung der Tonizität ein Alkali- oder Erdalkalimetallhalogenid, Dextrose, Xylit, Glucose, Mannit oder Sorbit ist.

3. Zusammensetzung nach Anspruch 1 oder 2, die zusätzlich ein physiologisch aktives Mittel enthält.

4. Zusammensetzung zur Verwendung als ein Medikament, enthaltend ein physiologisch aktives Mittel, ausgewählt aus einem oder mehreren Arzneimitteln oder einem oder mehreren diagnostischen Mitteln, und
i) 0,1 bis 10 Gew.-% eines Mittels zur Einstellung der Tonizität, bezogen auf das Gesamtgewicht der Zusammensetzung,
ii) ein flüssiges Verdünnungsmittel, von dem wenigstens 55 Gew.-% Wasser sind, und
iii) 0,1 bis 6 Gew.-% eines Celluloseethers, bezogen auf das Gesamtgewicht der Zusammensetzung,
wobei der Celluloseether eine Viskosität von 1,2 bis 8000 mPa·s, gemessen als eine 2-gew.-%ige wässrige Lösung bei 20°C und einer Scherrate von 10 s⁻¹, aufweist und
worin der Celluloseether Anhydroglucoseeinheiten, die über 1-4-Verknüpfungen miteinander verbunden sind, aufweist, und Methylgruppen, Hydroxyalkylgruppen und optional Alkylgruppen, die sich von Methyl unterscheiden, als Substituenten aufweist, so dass
die Hydroxylgruppen der Anhydroglucoseeinheiten mit Methylgruppen substituiert sind, so dass s23/s26 gleich 0,29 oder kleiner ist,
wobei s23 der Molenbruch der Anhydroglucoseeinheiten ist, bei denen nur die zwei Hydroxylgruppen in den 2- und 3-Positionen der Anhydroglucoseeinheiten mit einer Methylgruppe substituiert sind, und
wobei s26 der Molenbruch der Anhydroglucoseeinheiten ist, bei denen nur die zwei Hydroxylgruppen in den 2- oder 6-Positionen der Anhydroglucoseeinheit mit einer Methylgruppe substituiert sind.

5. Zusammensetzung nach einem der Ansprüche 1 bis 4, wobei der Celluloseether eine Viskosität von 1,8 bis 6000 mPa·s, gemessen als eine 2-gew.-%ige wässrige Lösung bei 20°C bei einer Scherrate von 10 s⁻¹, aufweist.

6. Zusammensetzung nach einem der Ansprüche 1 bis 5, wobei der Celluloseether eine Viskosität von 2,4 bis 1000 mPa·s, gemessen als eine 2-gew.-%ige wässrige Lösung bei 20°C bei einer Scherrate von 10 s⁻¹, aufweist.

7. Zusammensetzung nach einem der Ansprüche 1 bis 6, wobei der Celluloseehter ein MS(Hydroxyalkyl) von 0,05 bis 0,35 aufweist.

8. Zusammensetzung nach einem der Ansprüche 1 bis 7, enthaltend 0,2 bis 8,0 Gew.-% eines Mittels zur Einstellung der Tonizität, bezogen auf das Gesamtgewicht der Zusammensetzung.

9. Zusammensetzung nach einem der Ansprüche 1 bis 8, die eine Viskosität von 2,4 bis 8000 mPa·s, gemessen bei 5°C und bei einer Scherrate von 10 s⁻¹, aufweist.

10. Zusammensetzung nach einem der Ansprüche 1 bis 9, die eine Gelbildungstemperatur von 18 bis 37°C aufweist.

11. Zusammensetzung nach einem der Ansprüche 1 bis 10 für die intranasale Verabreichung.

12. Zusammensetzung nach einem der Ansprüche 1 bis 11, enthaltend 0,1 bis 6 Prozent des Celluloseethers, 0,1 bis 10 Prozent des Mittels zur Einstellung der Tonizität, 0 bis 20 Prozent eines physiologisch aktiven Mittels und 0 bis 30 Prozent eines oder mehrerer optionaler Hilfsstoffe, bezogen auf das Gesamtgewicht der Zusammensetzung, wobei der Rest das flüssige Verdünnungsmittel ist.

13. Zusammensetzung nach einem der Ansprüche 3 bis 12, wobei das physiologisch aktive Mittel ausgewählt ist aus einem oder mehreren Arzneimitteln, einem oder mehreren diagnostischen Mitteln, einem oder mehreren essentiellen Ölen, einem oder mehreren physiologisch aktiven Mitteln, die für kosmetische oder Ernährungszwecke geeignet sind.

14. Ein Behälter, enthaltend die Zusammensetzung nach einem der Ansprüche 1 bis 13, wobei der Behälter so gestaltet ist, um die Zusammensetzung durch Sprühen oder als Tropfen freizugeben.

15. Zusammensetzung nach einem der Ansprüche 3 bis 13 zur Verwendung in der transmucosalen Verabreichung eines physiologisch aktiven Mittels an ein Individuum, wobei diese Zusammensetzung auf eine Schleimhaut des Individuums aufgebracht wird.

## Revendications

1. Une composition pour une application sur une muqueuse comprenant
i) de 0,1 à 10 pour cent en poids d'un agent d'ajustement de tonicité, rapporté au poids total de la composition,
ii) un diluant liquide dont au moins 55 pour cent en poids sont de l'eau, et
iii) de 0,1 à 6 pour cent en poids d'un éther de cellulose, rapporté au poids total de la composition,
dans laquelle l'éther de cellulose a une viscosité allant de 1,2 à 8 000 mPa•s, mesurée sous la forme d'une solution aqueuse à 2 % en poids à 20 °C à une vitesse de cisaillement de 10 s⁻¹, et
dans laquelle l'éther de cellulose a des unités anhydroglucose unies par 1 à 4 liaisons et a des groupes méthyle, des groupes hydroxyalkyle, et facultativement des groupes alkyle qui sont différents d'un méthyle en tant que substituants de telle sorte que des groupes hydroxyle d'unités anhydroglucose soient substitués par des groupes méthyle de telle sorte que s23/s26 soit de 0,29 ou moins,
dans laquelle s23 est la fraction molaire d'unités anhydroglucose dans laquelle seuls les deux groupes hydroxyle dans les positions 2 et 3 de l'unité anhydroglucose sont substitués par un groupe méthyle et
dans laquelle s26 est la fraction molaire d'unités anhydroglucose dans laquelle seuls les deux groupes hydroxyle dans les positions 2 et 6 de l'unité anhydroglucose sont substitués par un groupe méthyle.

2. La composition de la revendication 1 dans laquelle l'agent d'ajustement de tonicité est un halogénure de métal alcalin ou alcalinoterreux, le dextrose, le xylitol, le glucose, le mannitol, ou le sorbitol.

3. La composition de la revendication 1 ou de la revendication 2 comprenant de plus un agent physiologiquement actif.

4. Une composition destinée à être utilisée en tant que médicament comprenant un agent physiologiquement actif sélectionné parmi un ou plusieurs produits médicamenteux ou un ou plusieurs agents diagnostiques et
i) de 0,1 à 10 pour cent en poids d'un agent d'ajustement de tonicité, rapporté au poids total de la composition,
ii) un diluant liquide dont au moins 55 pour cent en poids sont de l'eau, et
iii) de 0,1 à 6 pour cent en poids d'un éther de cellulose, rapporté au poids total de la composition,
dans laquelle l'éther de cellulose a une viscosité allant de 1,2 à 8 000 mPa•s, mesurée sous la forme d'une solution aqueuse à 2 % en poids à 20 °C à une vitesse de cisaillement de 10 s⁻¹, et
dans laquelle l'éther de cellulose a des unités anhydroglucose unies par 1 à 4 liaisons et a des groupes méthyle, des groupes hydroxyalkyle, et facultativement des groupes alkyle qui sont différents d'un méthyle en tant que substituants de telle sorte que des groupes hydroxyle d'unités anhydroglucose soient substitués par des groupes méthyle de telle sorte que s23/s26 soit de 0,29 ou moins,
dans laquelle s23 est la fraction molaire d'unités anhydroglucose dans laquelle seuls les deux groupes hydroxyle dans les positions 2 et 3 de l'unité anhydroglucose sont substitués par un groupe méthyle et
dans laquelle s26 est la fraction molaire d'unités anhydroglucose dans laquelle seuls les deux groupes hydroxyle dans les positions 2 et 6 de l'unité anhydroglucose sont substitués par un groupe méthyle.

5. La composition de n'importe laquelle des revendications 1 à 4 dans laquelle l'éther de cellulose a une viscosité allant de 1,8 à 6 000 mPa•s, mesurée sous la forme d'une solution aqueuse à 2 % en poids à 20 °C à une vitesse de cisaillement de 10 s⁻¹.

6. La composition de n'importe laquelle des revendications 1 à 5 dans laquelle l'éther de cellulose a une viscosité allant de 2,4 à 1 000 mPa•s, mesurée sous la forme d'une solution aqueuse à 2 % en poids à 20 °C à une vitesse de cisaillement de 10 s⁻¹.

7. La composition de n'importe laquelle des revendications 1 à 6 dans laquelle l'éther de cellulose a une SM(hydroxyalkyle) de 0,05 à 0,35.

8. La composition de n'importe laquelle des revendications 1 à 7 comprenant de 0,2 à 8,0 pour cent en poids d'un agent d'ajustement de tonicité, rapporté au poids total de la composition.

9. La composition de n'importe laquelle des revendications 1 à 8 ayant une viscosité allant de 2,4 à 8 000 mPa•s, mesurée à 5 °C et à une vitesse de cisaillement de 10 s⁻¹.

10. La composition de n'importe laquelle des revendications 1 à 9 présentant une température de gélification allant de 18 à 37 °C.

11. La composition de n'importe laquelle des revendications 1 à 10 pour une administration intranasale.

12. La composition de n'importe laquelle des revendications 1 à 11 comprenant de 0,1 à 6 pour cent de l'éther de cellulose, de 0,1 à 10 pour cent d'un agent d'ajustement de tonicité, de 0 à 20 pour cent d'un agent physiologiquement actif, et de 0 à 30 pour cent d'un ou de plusieurs adjuvants facultatifs, rapporté au poids total de la composition, le reste étant le diluant liquide.

13. La composition de n'importe laquelle des revendications 3 à 12 dans laquelle l'agent physiologiquement actif est sélectionné parmi un ou plusieurs produits médicamenteux, un ou plusieurs agents diagnostiques, une ou plusieurs huiles essentielles, ou un ou plusieurs agents physiologiquement actifs qui sont utiles à des fins cosmétiques ou nutritionnelles.

14. Un récipient comprenant la composition de n'importe laquelle des revendications 1 à 13, le récipient étant conçu pour libérer la composition par pulvérisation ou sous forme de gouttes.

15. La composition de n'importe lesquelles des revendications 3 à 13 pour l'utilisation dans l'administration transmucosale d'un agent physiologiquement actif chez un individu, ladite composition étant appliquée sur une muqueuse de l'individu.
